# EUROPEAN PATENT SPECIFICATION

(11) **EP 2 111 866 B1**
(45) Date of publication and mention of the grant of the patent: **17.10.2012**
(21) Application number: 09405061.4
(22) Date of filing: 03.04.2009
(51) Int. Cl.: A61K 36/185, A61K 9/20, A61P 17/14

(54) **Pharmaceutical oral compositions obtained by hydroalcoholic extraction of Boehmeria nipononivea plants titrated in rutin and fatty acids with anhydrous colloidal silica**
Pharmazeutische Zusammensetzungen zur oralen Verwendung gewonnen durch wässrig-alkoholische Extraktion der Pflanze Boehmeria nipononivea in Rutin und Fettsäuren unter Zugabe von wasserfreiem kolloidalem Siliciumdioxid titriert
Compositions pharmaceutiques pour utilisation orale obtenuées par extraction hydro-alcoolique en présence de dioxyde de silice colloïdale anhydre, à partir de plantes Boehmeria nipononivea et titrées en rutine et acides gras

(30) Priority: 07.04.2008 CH 540082008
(43) Date of publication of application: 28.10.2009
(73) Proprietor: Ancora, Monica, 6500 Bellinzona (CH)
(72) Inventor: Ancora, Monica, 6500 Bellinzona (CH)

(56) References cited:
- WO-A-2005/084621
- RINALDI F. ET AL.,: "The effect of herbal extract on hair growth in female androgenic alopecia" AMERICAN ACADEMY OF DERMATOLOGY. 63RD ANNUAL MEETING 18-23 FEBRUARY 2005, NEW ORLEANS, [Online] - February 2005 (2005-02) page 1, XP002544142 INTERNET Retrieved from the Internet: URL:http://www.valxer.it/Valxer/1006008/Po ster.pdf>
- SHIMIZU K ET AL: "Steroid 5alpha-Reductase Inhibitory Activity and Hair Regrowth Effects of an Extract from Boehmeria nipononivea" BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 64, no. 4, 1 January 2000 (2000-01-01), pages 875-877, XP002333116 ISSN: 0916-8451
- DATABASE WPI Week 199814 Thomson Scientific, London, GB; AN 1998-148349 XP002544143 & JP 10 017456 A (KANSAI KOSO KK) 20 January 1998 (1998-01-20)

## Description

### Introduction

The Boehmeria nipononivea or B. niponivea Koidz is also known as Kara Musi or Karamushi or Mushi Kogan and belongs to the family of Urticacae. It is a perennial plant originally from Japan that grows on wooded land to a height of 1.5 m with leaves similar to those Urticae plants. The leaves are green on top and white on the bottom. In folk medicine it is used for problems of the liver and as a diuretic. The Boehmeria niponivea is a subspecies of Boehmeria nivea. Among the species there are the B.cylindrica, B. japonica, B.macrophylla, B.malabaric, B. platyphylla, B.spicata, B.tricuspiss, and B. nivea, which is known as ramie or Ramia and is used for the production of textile fibres.

Studies have been performed on B. niponivea to determine the capacity of the regrowth of hair on mice. They identified and quantified through extract of the plant in acetone , the polyunsaturated fatty acids such as α-linolenic acid, linoleic, palmitic, elaidic, oleic and stearic which have inhibitory activity on the enzyme 5 α-reductase. The quantities of these fatty acids have been compared with other species of plants and it looks like that B. nipononivea is a plant with higher values of these components ( about 15% in weight in the acetone extract).

### Chemical characteristics, organoleptic, physical dry

The part of the plant to be used includes the derial part.
If necessary, the medium in which you can 'join the plant consists of maltodextrins USP.

The solvent extraction is made up of 30% ethyl alcohol in distilled water 70.
The extraction ratio should be between 15-30:1 (preferably by spray dry method).

The appearance of the plant should be light brown-brown, with a characteristic smell and taste (taste test). The assay of the fatty acids should range between 10-33% (HPLC method or UVvis). The mesh size obtained with 80 mesh should be between 80-100 mesh. Density (bulk density) must be between 0.4-0.8 gm / ml (method USP23), while the tapped density must be between 0.5-0.95 gm / ml (23 USP method). Solubility should be good both in water and in alcohol (IP). The pH in solution of 1% should be between 6-8 where the extraction ratio is 15:1, or between 3.5-5 if the ratio is 30:1. The humidity must be less than 5 and titrate rutina must exceed 5%. The plant must be free of heavy metals, pesticides, genetically modified organisms, aflatoxins, yeasts, molds, Escherichia coli, staphylococci and other microorganisms possible.

### Method of processing

The B. niponivea must be mixed in the presence of anhydrous colloidal silica commercially known as Aerosil 200, its specific surface area can vary from 50 to 450 m2 / g with a particle diameter of between 10 and 40 nm, 7:2 in weight ratio expressed as grams for encapsulation of 60 hard gelatin capsules of type 0.
Each capsule of type 0 must contain 116 milligrams of dry extract and 33 mg of silica colloidal anhydrous.
Mixing must be approved by a mixer for mixing of powders for use with medical rpm minimum (intensity 1) per minute not exceeding 20,000, and the time of mixing should be 15-18 minutes. The mixture should appear homogeneous, there must be flocculate the colloidal anhydrous silica. With the presence of silica the smell of B. nipononivea should not be intense. The color should be uniform.
The encapsulation must be hard operculum type 0, with density of about 0.68 ml measured with graduated cylinder and capsules for total dust by encapsulating equal to about 150 mg of powder from a single capsule encapsulating. The variation depends on the density of the mixture of dust and moisture by encapsulating dry. Always be aware that the final weight of the capsule must respond to the control of uniformity of mass provided by the various pharmacopoeias.
The dosage of the formulation is 2-3 capsules per day to be taken either before, during or after meals.

### Object of the invention

The invention pertains specifically to the production of tablets and capsules obtained from hydroalcoholic extraction of Boehmeria nipononivea or B. niponivea Koidz for oral use. The present invention aims to develop a safe product to be administered orally allowing the regrowth of hair bulbs. It increases the bone marrow density of the hair follicle , and has activities on androgenetic alopecia. The action is directed primarily on the enzymatic system steroid 5 α reductase.

### Exposure of the invention:

### State of the art

Androgenic alopecia strikes throughout life around 80% of men and 50% of women.

Androgenic alopecia is characterized by the structural miniaturization of the follicles of the hair in androgen sensitive susceptible individuals, and is anatomically defined for a certain type of scalp.

Biochemically, a predisposing factor of this disorder -further to that genetic -, is that of the conversion of testosterone in dihydrotestosterone (DHT) by the enzymatic system of 5 α reductase.

It would seem that the presence of the level DHT of the hair follicles is harmful for the hair bulbs and is harmful for the hair.

This metabolism is also the key for the commencement and the progression of the benign prostatic hyperplastic. Many medicines used for this pathology have given unexpected results on the androgenic alopecia.

In a controlled pilot study, with males between the 23 and 64 years with light or moderate androgenic alopecia it was observed that the drawn out liposterolic of Serenoa repens and β-sitosterol brought an improvement of the alopecia in the 60% of the patients essayed.

This study has confirmed for the first time the possible efficacy of the inhibitors of the via enzymatic 5 α reductase to treat baldness.

Studies of enzymatic competition in vitro on the enzymatic 5 α reductase confirm certain polyunsaturated acids (α,γ-linolenic) as able to inhibit the activity of this enzyme.

The extract of B. nipononivea is a powerful activity inhibitor of the enzyme 5 α reductase and promotes the regrowth of hair on mice.

The activity on the enzyme 5 α reductase is attributed to the presence of six fatty acids (α linolenic, linoleic, palmitic and in least quantity elaidic, oleic and stearic acid), while the activity of the extract of B. nipononivea on the regrowth of hair has also been attributed to the presence of other phyto elements no identified of non androgen-dependent action.
Previous studies have verified that in the fractions of extracts of B.nipononivea the α linolenic, linoleic and oleic acids contribute to inhibit the enzyme 5 α reductase.

It is important to notice that the animal model used for the experiment does not have an androgen-dependent balding and it has previously been measured only in connection to the regrowth of trimmed hair.

The reason for the induction of hair growth in mice has been attributed in particular to the α-linolenic, stearic and elaidic and, acids due to fatty acid content of inhibitors of the enzymatic system of 5 α reductase in B.nipononivea favours this plant to support the regrowth of hair.

Background Art and disadvantages

To date products have been used that increase the local vascularisation based on hormonal vasodilators, hormones, or genuine products with mixtures of food supplements and herbal plants to encourage action. Except for the synthetic products with hormonal activity (such as finasteride, minoxidil on the spironolactone and progesterone, and others which have significant and scientifically proven side effects in general decrease in libido, dysfunction and disorder premature erection , problems of fetal malformations in pregnant women, etc.), the various mixtures of plants and trace elements, vitamins or other supplements have failed to focus on local action leading to lasting regrowth of hair and hair bulbs.

Rinaldi et al. 2005 (American Academy of Dermatology. 63rd Annual Meeting 18-23 February 2005, New Orleans) disclose the use of an extract of B. nipononivea which inhibits the activity of 5-alpha-reductase. The efficacy of the extract was tested in women affected by androgenic alopecia by administration of 200 mg of a B. nippononivea extract (one tablet once a day), resulting in reduction of hair miniaturization, an improvement of anagen hair or anagen/telogen ratio, and hair regrowth stimulation. This document is silent about the amount of polyunsaturated fatty acids and rutin present in the extract.

Patent application WO 2005/084621 A 1 discloses oral pharmaceutical compositions and food supplements containing 100-200 mg B. nipononivea extract which inhibit the 5-alpha-reductase enzyme. The extract contains a low concentration of polyunsaturated fatty acids (2-8%) but there is no mention about the presence of rutin. The compositions are useful in the treatment or prevention of androgenic alopecia, acne, seborrhea, hypertrichosis, hirsutism and/or telogenic effluvium.

### Solution

The aim is achieved thanks to the characteristics of claim 1.
The dependent claims reveal advantageous developments: the product has activity on the alopecia androgenetic activity over the long-term due to the inhibitory action on steroid enzyme 5 α reductase locally only on the scalp, without side effects, toxicity and precaution known to date.

### Bibliografia

1. http:// www.calvizie.net
2. http://www.ibiblio.org/pfaf/gibin/arr_html?Boehmeria+nipononivea&CAN=LATIND
3. Britton. N. L. Brown. A. An Illustrated Flora of the Northern United States and Canada DoverPublications. New York. 1970 ISBN 0-486-22642-5 Reprint of a 1913 Flora, but still a very useful book.
4. Ohwi. G. Flora of Japan. (English translation) Smithsonian Institution 1965
5. Asiatica International Rare Plant Resource http://asiaticanursery.com/product_info.php/products_id/698
6. http://www.homolaicus.com/scienza/erbario/utility/botanica_sistematica/hypertext/1540.htm
7. Agricultural Research Service http://www.arsgrin.gov/cgibin/duke/ethnobot.pl?ethnobot.taxon=Boehmeria%20nipononivea
8. Kunkel. G. Plants for Human Consumption. Koeltz Scientific Books 1984 ISBN 3874292169 An excellent book for the dedicated. A comprehensive listing of latin names with a brief list of edible parts.
9. The Global Compendium of Weeds http://www.hear.org/gcw/html/autogend/species/2821.HTM
10. Krmpotic E, Farnsworth NR, Messmer WM. Cryptopleurine, an active antiviral alkaloid from Boehmeria cylindrica (L.) Sw. (Urticaceae). J Pharm Sci. 1972 Sep;61 (9):1508-9.
11. Takemoto T, Miyase T. [Studies on the constituents of Boehmeria tricuspis Makino. II (author's transl)] [Article in Japanese]Yakugaku Zasshi. 1975 Feb;95(2):180-4.
12. Bruhlmann F, Leupin M, Erismann KH, Fiechter A. Enzymatic degumming of ramie bast fibers.Biotechnol. 2000 Jan 7;76(1):43-50.
13. Shimizu K, Kondo R, Sakai K, Shoyama Y, Sato H, Ueno T. Steroid 5alpha-reductase inhibitory activity and hair regrowth effects of an extract from Boehmeria nipononivea. Biosci Biotechnol Biochem. 2000 Apr;64(4):875-7
14. http://www.lapelle.it/dermatologia/calvizia.htm
15. Prager N, Bickett K, French N, Marcovici G. A randomized, double-blind, placebo-controlled trial to determine the effectiveness of botanically derived inhibitors of 5-alpha-reductase in the treatment of androgenetic alopecia. J Altern Complement Med. 2002 Apr;8(2):143-52.
16. Liang T, Liao S. Inhibition of steroid 5 alpha-reductase by specific aliphatic unsaturated fatty acids.Biochem J. 1992 Jul 15;285 (Pt 2):557-62.
17. Pham H, Ziboh VA.5 alpha-reductase-catalyzed conversion of testosterone to dihydrotestosterone is increased in prostatic adenocarcinoma cells: suppression by 15-lipoxygenase metabolites of gamma-linolenic and eicosapentaenoic acids. J Steroid Biochem Mol Biol. 2002 Nov;82(4-5):393-400.
18. Buhl AE, Waldon DJ, Baker CA, Johnson GA,Upjohn Company, Kalamazoo, Michigan 49001. Minoxidil sulfate is the active metabolite that stimulates hair follicles. J Invest Dermatol, 95: 5, 1990 Nov, 553-7
19. Zappacosta AR: Reversal of baldness in a patient receiving minoxidil for hypertension. N Engl J Med 303:1480-1481, 1980
20. Olsen EA, Weiner MS, Delong ER, et al: Topical minoxidil in early male pattern baldness. J Am Acad Dermatol 82:90-93, 1984
21. Weiss VC, West DP, Mueller CE: Topical minoxidil in alopecia areata. J Am Acad Dermatol 5:224-226, 1981
22. Weiss VC, Uno H, Buys CM, et al: Histologic and immunopathic profiles in alopecia totalis patients receiving topical minoxidil (1% and 5%). J Invest Dermatol 84:360, 1985
23. Tosti A, Bardazzi F, DePadova MP, et al: Contact dermatitis due to minoxidil. Contact Derm 13:275-276, 1985
24. Friedman ES, Friedman PM, Cohen DE, Washenik K. Ronald O. Perelman Department of Dermatology, New York University School of Medicine, New York, NY, USA. Dermatite allergica da contatto dovuta a soluzioni topiche di minoxidil: eziologia(branca della scienza che studia l'origine delle malattie) e trattamento.J Am Acad Dermatol. 2002 Feb;46(2):309-12. Related Articles, Links PMID: 11807448 [PubMed - indexed for MEDLINE]
25. J Rundegren, R Cuddihy, JR Spindler, R Trancik. Prevention or Stabilization of Hair Loss with Minoxidil Topical Solution: Can It Be Quantitated? Presented at the 60th American Academy of Dermatology (AAD) Annual Meeting February 2002**.**
26. F.Bettiol, Manuale delle preparazioni galeniche, Tecniche nuove 1996
27. The Japanese Pharmacopeia XV editino,**2006**
28. P. Jäger, Formulario Galenico Europeo, Edioptima edizione, 2005
29. G. Proserpio, La Neogalenica,dalla galenica tradizionale alla neogalenica nelle preparazioni topiche, OEMF ed.1995
30. M. Amorosa, Principi di tecnica farmaceutica, Libreria Universitaria L.Tinarelli Bologna, 1995
31. Rinaldi et al.,: "The effect of herbal extract on hair growth in female androgenic alopecia" American Academy of Dermatology. 63rd Annual Meeting 18-23 February 2005, New Orleans, (URL:http://www.valxer.it/Valxer/1006008/Poster.pdf)
32. WO 2005/084621 A1 (Giuliani SPA [IT]; Giuliani, G. [IT]; Benedusi, A.[IT]; Bellinvia, S. [IT] and Rinaldi, F. [IT]) 15 September 2005.

## Claims

1. Pharmaceutical oral composition as tablets or capsules obtained by hydroalcoholic extraction of Boehmeria niporonivea, whereby the assay of fatty acids should range between 10-33% (WPLC method or UV vis) and the titrated rutin must exceed 5%, and whereby the mixed in the presence be mixed in the presence of anhydrous colloidal silica

2. Pharmaceutical oral composition according to claim 1 **characterized by** a daily oral dose of about 350 mg of dry extract, where the capsule can be hard, soft, gastro-resistand or capsules of types 00,0 1, 2, 3, 4, 5, for single or multiple administration.

3. Pharmaceutical oral composition according to claim 1 or 2, whereby the extract in the capsules is present as dry form, only fluid or syrup.

4. Pharmaceutical oral composition according to claim 1, 2, or 3, where the dry extract contains other insert ingredients.

5. Pharmaceutical oral composition according to any of the previous claims, whereby the tablets are prepared by direct composition of the dry extract, optionally in the presence of inert ingredients.

6. Pharmaceutical oral composition according to any of the previous claims, further comprising other kinds of plants, such as Bochmeria platanifolia or sieboldiana.

7. Pharmaceutical oral composition according to any of the previous claims, comprising as additives inert excipients, lubricants, adsorbents, surfactants, glidants, suspending agents, Thickness, viscosity including agents, dryes... substances that generate effervescence, disintegrators, lactose and/or spray dried lactose, magnesium stearate, microcrystalline cellulose, Talc, calcium stearate, magnesium stearate, calcium carbonate, corn starch, kaolin, clay, pregelatinized starch, aluminium silicate or magnesium.

8. Pharmaceutical oral composition according to any of the previous claims for use as food supplement.

9. Pharmaceutical oral composition according to any of the previous claims, whereby the aerial parts of the plant are used

10. Pharmaceutical oral composition according to any of the previous claims for use in the treatment of androgenetic alopecia, hair fall or scalp trophism.

11. Phamaceutical oral composition according to any of the previous claims, whereby the final formulation is a cachet, a sachet or a sublingual form.

## Patentansprüche

1. Die orale pharmazeutische Zusammensetzung der Tabletten oder Kapseln kommt durch hydroalkoholische Extraktion der Pflanze Boehmeria nipononivea zustande wobei der Fettsäuregehalt zwischen 10-33% liegen sollte (Methode WPhC oder UVsis) und die Betitelung von Rutin um 5% grösser sein muss und damit wird die Bohemeria nipononivea im Vorhandensein von anhydro colloidal silica gemischt.

2. Die orale pharmazeutische Zusammensetzung gemäß Anforderung 1 ist von der täglichen Dosis von ungefähr 350mg Trockenextrakt charakterisiert, wobei es sich dabei um harte, weiche, magensaftresistente Kapseln handeln kann oder um Kapseln des Typ 00,0,1,2,3,4,5, geeignet für eine individuelle und vielfältige Anwendung.

3. Bei der oralen pharmazeutischen Zusammensetzung gemäß Anforderung 1 oder 2 präsentiert sich das Extrakt in den Kapseln in trockener Form, öliger Flüssigkeit oder Sirup.

4. Bei der oralen pharmazeutischen Zusammensetzung gemäß Anforderung 1,2, oder 3 enthält das Trockenextrakt auch noch andere Hilfsstoffe.

5. Bei der oralen pharmazeutischen Zusammensetzung gemäß all den vorgenannten Anforderungen werden die Tabletten durch direkte Kompression des Trockenextrakts in Gegenwart von Hilfsstoffen hergestellt.

6. Die orale pharmazeutische Zusammensetzung gemäß all den vorgenannten Anforderungen enthält auch andere Pflanzenarten wie die Boehmeria platanifolia oder sieboldiana.

7. Die orale pharmazeutische Zusammensetzung gemäß all den vorgenannten Anforderungen enthält auch noch andere Zutaten: Hilfsstoffe, Schmierstoffe, Adsorbentien, Tenside, Gleitmittel, Suspendiermittel, Verdickungsmittel, Viskosität induzierende Wirkstoffe, Trockenmittel, Substanzen die Aufbrausen produzieren, Sprengmittel, Laktose und/oder Laktose sprühgetrocknet, Magnesiumstearat, mikrokristalline Cellulose, Talkum, Calciumstearat, Kalziumcarbonat, Maisstärke, Kaolin, Ton, Quellstärke, Aluminium- oder Magnesium Silikat.

8. Die orale pharmazeutische Zusammensetzung gemäß all den vorgenannten Anforderungen wird als alternatives nahrungsergänzendes Lebensmittel verwendet.

9. Für die orale pharmazeutische Zusammensetzung gemäß all den vorgenannten Anforderungen werden die oberirdischen Teile der Pflanze verwendet.

10. Die orale pharmazeutische Zusammensetzung gemäß all den vorgenannten Anforderungen wird bei Behandlung von androgenetischer Alopezie, Haarverlust oder Kopfhaut Trophik angewendet.

11. Die orale pharmazeutische Zusammensetzung gemäß all den vorgenannten Anforderungen präsentiert sich in der endgültigen Struckturformel als Tablette, als Beutel oder in sublingualer Form.

## Revendications

1. La composition pharmaceutique orale des comprimés ou capsules obtenues par l'extraction hydro-alcoolique du Boehmeria nipononivea, où le contenu des acides gras devrait ranger entre 10-33% (méthode WPhC ou UVsis) et la titré en rutine doit excéder le 5% et par lequel le B.nipononivea doit être mélangé dans la présence de silice colloïdal anhydre.

2. La composition pharmaceutique orale selon le claim 1 est **caractérisée par** la dose orale quotidienne de à peu près 350mg d'extrait sec, où la capsule peut être dure, mou, gastro-résistante ou capsules de type 00, 0,1,2,3,4,5 pour une administration individuelle ou multiple.

3. La composition pharmaceutique orale selon le claim 1 ou 2, l'extrait dans les capsules est présent sous forme sèche, fluide huileux ou sirop.

4. La composition pharmaceutique orale selon les claims 1, 2 ou 3, l'extrait sec contient autres ingrédients inertes.

5. La composition pharmaceutique orale selon touts les claims d'auparavant, les comprimés sont préparées par la compression directe de l'extrait sec, facultativement dans la présence d'ingrédients inertes.

6. La composition pharmaceutique orale selon touts les claims d'auparavant, contient aussi autres types de plantes, comme la Boehmeria platanifolia ou sieboldiana.

7. La composition pharmaceutique orale selon touts les claims d'auparavant, contient autres additives : excipients inertes, lubrifiants,adsorbants, des surfactants, des agents glissants, des agents de suspension, des agents épaississants, des agents induisant la viscosité, agents de séchage, substances qui produisent effervescence, désagrégeant, lactose et/ou lactose aérosol(spray) sec, magnesium stearate, cellulose microcristalline, talc, stéarate de calcium, stéarate de magnésium, carbonate de calcium, amidon de maïs, le kaolin, argile, amidon prégélatinisé, de l'aluminium silicate ou magnesium.

8. La composition pharmaceutique orale selon touts les claims d'auparavant est à utiliser comme suppléant alimentaire.

9. La composition pharmaceutique orale selon touts les claims d'auparavant utilise les parties aériennes de la plante.

10. La composition pharmaceutique orale selon touts les claims d'auparavant à utiliser dans le traitement de l'alopecia androgénétique, perte de cheveux ou trophisme du cuir chevelu.

11. La composition pharmaceutique orale selon touts les claims d'auparavant, par laquelle la formulation finale est un cachet, un sachet ou sous la forme sublinguale.
